# EUROPEAN PATENT APPLICATION

(11) **EP 1 553 099 A1**
(43) Date of publication of application: **13.07.2005**
(21) Application number: 03766107.1
(22) Date of filing: 29.07.2003
(51) Int. Cl.: C07D 475/14, A61K 31/525, A61P 1/02, A61P 1/04, A61P 9/10, A61P 9/12, A61P 17/02, A61P 19/02

(54) **A RIBOFLAVIN DERIVATIVE AND ITS MANUFACTURE AND USES**

(30) Priority: 02.08.2002 CN 02125917
(71) Applicant: Institute of Radiation Medicine Academy of Military Medical Sciences of the Pla, Beijing 100850 (CN); Shenzhen Salubris Pharmaceuticals Co., Ltd., Shenzhen 518026 (CN)
(72) Inventor: XU, Qishou, Haidian District, Beijing 100850 (CN); WANG, Lin, Haidian District, Beijing 100850 (CN); TONG, Chaoyang, Haidian District, Beijing 100850 (CN); GUO, Changjiang, Haidian District, Beijing 100850 (CN); LU, Qiujun, Haidian District, Beijing 100850 (CN); YUN, Liuhong, Haidian District, Beijing 100850 (CN); DAI, Lihua, Haidian District, Beijing 100850 (CN); SUN, Mingtang, Haidian District, Beijing 100850 (CN)
(74) Representative: Ebner von Eschenbach, Jennifer
(86) International application number: PCT/CN2003/000609
(87) International publication number: WO 2004/013142

(57) **Abstract**

The invention discloses riboflavin derivatives, and a process of preparing the same, and their applications. The water in oil suspending preparation of the riboflavin derivatives has high stability, and the effects could last for 3 months after intramuscular injection at dose of 150mg. This invention increases significantly the bioavailability of riboflavin *in vivo,* and provides an important treatment method for cure of ariboflavinosis. The preparation is found to have remarkable effect on dermatitis, oral and digestive tract catarrhs caused by bone marrow transplantation for leukemia and chemotherapy for tumor. It is also found to have noticeable effect on persistent oral ulcer, and enhance the tolerability to chemotherapy and radiotherapy. In the treatment of coronary heart disease and hypertension syndrome, arthritis and burn wound, the preparation also has significant effects. This invention has a wide application perspective for its simple techniques, definite long-acting action and effectiveness.

## Description

### FIELD OF THE INVENTION

The present invention relates to riboflavin derivatives, a process for preparation of the same, and their applications.

### BACKGROUND OF INVENTION

Riboflavin, such as, the coenzyme of the flavin-enzymes, participates in the complex oxidation reactions in organisms and possesses important pharmacological effects. Recent pharmacological studies indicate that the riboflavin shows certain pharmacological effects in the cure of coronary heart disease, hypertension syndrome, and heartburn besides traditionally treating ariboflavinosis. However, the riboflavin is a water-soluble vitamin which is easy to be excreted in urine and sweat, leading to low bioavailability and short half-time. Therefore, people are susceptible to ariboflavinosis, such as buccal-genital syndrome (cheilitis, glossitis, angular cheilitis and scrotum dermatitis), conjunctivitis and intermediate uveitis which influence the vision when the riboflavin intake decreases or its requirement increases. For the prevention and treatment of ariboflavinosis, many studies focused on synthesizing appropriate riboflavin derivatives as pro-drug of the riboflavin which could slowly release the riboflavin and the availability of riboflavin could be enhanced significantly *in vivo.* Japanese researchers Kunio, Miyamoto *et al* synthesized the tetra-palmitate ester, tetra-decanoate ester, tetra-butyrate ester, tetra-acetate ester and tetra-tryptophan ester of riboflavin in pyridine using riboflavin and fatty acid chloride/ acid anhydride. However, these esters could not be hydrolyzed in saliva or intestinal fluid. Furthermore, studies on the tetra-palmitate of riboflavin indicated that it did not have any biological activity of riboflavin.

### SUMMARY OF THE INVENTION

An object of the present invention is to provide riboflavin derivatives, and a process of preparation thereof. Such a derivative contains long-acting group, which can help to enhance the availability of riboflavin *in vivo* by increasing the retention, absorption, bioavailability, and delay of the riboflavin excretion.

This invention involves all ester derivatives of riboflavin or hydroxyl group of ribitol on the lateral chain of sulforiboflavin except the tetra-stearate ester, tetra-palmitate ester, tri-laurate ester, tetra-decanoate ester, tetra-butyrate ester, tetra-acetate ester, tetra-tryptophan ester, and phosphate ester of riboflavin.

The described riboflavin derivatives are the mono-, di-, tri- or tetra-esters of the compound of Formula (I), in which the groups of R₁, R₂, R₃, R₄ may be different, identical or independent to each other.

The riboflavin derivatives described above can all be prepared with general methods.

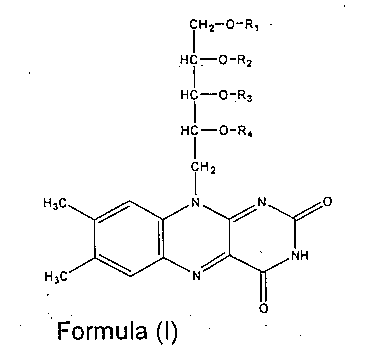

The preferred compound of the present invention is the 5'-lauric acid monoester of riboflavin of Formula (II).

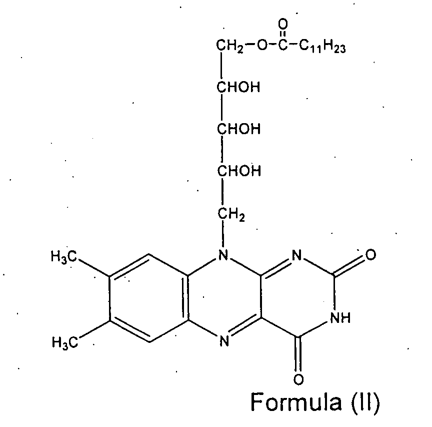

The 5'-laurate monoester of riboflavin was prepared by adding lauroyl chloride in riboflavin or sulforiboflavin suspending in polar organic solvent.

The preferred polar organic solvent for suspending riboflavin or sulforiboflavin is pyridine or N,N-dimethylformamide.

Another object of the present invention is to provide a long-acting preparation in which the active substance is the compound of the invention, and a process for production thereof.

A long-acting w/o suspending preparation of riboflavin is mainly composed of the compound of Formula II and ethyl oleate.

To enhance the fluidity, camellia oil was added into the w/o suspending preparation. The ratio of weight and volume of each ingredient is as following:

| | |
|---|---|
| Compound II | 50-150 mg |
| Ethyl oleate | 0.1-1 ml |
| Camellia oil | 0-1 ml |

The preferable ratio of weight and volume of each ingredient is:

| | |
|---|---|
| Compound of Formula II | 150 mg |
| Ethyl oleate | 0.5 ml |
| Camellia oil | 0.5 ml |

In this invention, the long-acting w/o suspending preparation of riboflavin was unified by conventional method after comminuting of materials.

Animal experiments proved that the riboflavin derivatives of the present invention showed a remarkable effect in treating ariboflavinosis and overcame the easy excretion from body fluid, short half-time and low bioavailability of current riboflavin drugs.

Studies on the structure activity and pharmacological mechanism of riboflavin showed that 5'-lauric acid monoester of riboflavin is the most high-yielded and effective compound in the invention. The compound, analyzed by four main spectroscopy methods (including 2-D NMR), was proved to be a new riboflavin derivative with definite structure. The long-acting w/o suspending preparation made from this compound could remain effective for three months after an intramuscular injection of 150mg. Therefore, it provides an important pharmaceutical agent for preventing and treating the general ariboflavinosis.

### BRIEF DESCRIPTION OF THE DRAWINGS

Figure 1 is a graph showing the change of food-intake of animals;
Figure 2 is a graph showing the change of body weight of animals;
Figure 3 is a graph showing the BGRAC change of animals; and
Figure 4 is a graph showing the change of riboflavin content in plasma.

### DETAILED DESCRIPTION OF THE INVENTION

The invention is further described by the following embodiments. However, the invention is not limited to these examples.

### Example I: Preparation of 5'-lauric acid monoester of riboflavin.

1200 ml pyridine, 1200 ml distilled water and 120 ml triethylamine were mixed and ice-bathed to maintain the inner temperature between 5-10°C. 25.0 g of riboflavin was then added and dissolved by electromagnetic stirring. The mixed solution was composed of lauroyl chloride and dioxane was added drop wisely in 25 min at 10°C. Then the mixture was stirred for 20 min. After lowering the inner temperature to 5°C by cryohydrate bath, the solution of 400 ml concentrated sulfuric acid and 600 ml distilled water was added drop wisely in 45 min, with temperature controlled between 10-15°C. The solution was then placed in ice water for 2.5 h, with the pH value of 6. After filtrated, washed by 2×100 ml water and pressed to dry, the dry product was then washed by 4×200ml diethyl ether and air-dried to gain 22.5 g primary product with the wash liquid discarded. The primary product was dissolved in concentrated hydrochloric acid and filtrated, followed by adding 50% ethanol. After sitting overnight, the solution was filtrated and the precipitated riboflavin was aired to be 20.5 g. The recovery yield was 82%. The mixture stock solution was evaporated under reduced pressure. Red powder was obtained and washed successively with ethyl ester, 5x100 ml dichloromethane and 5×50 ml methanol. The product was then aired, smashed and dried in a dryer containing P₂O₅ for 48 hours. The weight of the obtained red crude product of monoester of riboflavin was 2.60 g, with a yield of 38.9% (based on the recovered starting materials).

550g coarse silica gel of 100-200 meshes was loaded into a column. 10.45 g of the crude riboflavin monoester was immersed in 500ml mixing solvent of CH₂Cl₂:CH₃OH(95:5) and the supernatant was loaded onto the silica gel column. Then the column was immersed and loaded using the same mixing solvent until all crude samples were dissolved: The mobile phase of the solvent was used to elute. Twenty parts of eluted samples were collected and numbered (1)-(20). Each parts of eluted sample was 500ml. TLC analysis indicated that samples (1) and (2) contained monoesters and a small quantity of polyester, samples (3)-(16) were mainly composed of monoesters and samples (17)-(20) were lack of monoesters and had no other components. 4.08g of red solid was obtained after combining samples (3)-(20) and recovering the mixing solvent under 25-30°C. The red product was immersed respectively in 400ml Et₂O and 400ml Et₂O:CH₂Cl₂(4:1) for 8 hours. After filtrated and dried in a dryer containing P₂O₅, 3.00g of red product was obtained. Treated with the similar method, 0.24g of pure monoester product was obtained from the samples (1) and (2). The total final product was 3.24g and the yield of separation was 31.0%.

The final riboflavin monoester product showed homogeneity in HPLC with C₁₈ column and 97% methanol: 3% water as the mobile phase. The melting point of this compound is 249-250°C. The result of element analysis was C62.34,H7.58,N10.03 (calculated values,%) and C62.08,H7.74,N9.68 (measured values, %). The specific rotatory power is [α]²⁵_{D}=-8.4(C0.5,CH₂Cl₂:CH₃OH 1:1). UV(max,CH₃OH)225.8 nm,269.2nm,361.4nm,444.2nm.IR(cm⁻¹),3420(br,2 OH),
3178(OH),1728(br,2 C=O),1661(C=O). The molecular weight of the target compound is consistent to the peak in MS. ¹H-NMR(C₅D₅N),δ(ppm),8.19(s,1 H,Ar),7.89(s,1 H,Ar),5.55(m,1 H,HOCH),5.35(m,2H,OCH₂),5.10(m,1 H,HOCH),4.90(m,2 H,NCH₂),4.60(br,1 H,HOCH),
2.31(t,2 H,COCH₂),1.4(s,3 H,ArCH₃),2.15(s,3 H,ArCH₃),1.59(m,2 H,CH₃CH₂),0.83(t,3 H,CH₃CH₂), ¹³C-NMR(C₅D₅N), δ(ppm),173.9(C=O),160.91(C),159.9(C),146.6(C),
137.6(C), 136.2(C), 135.0(C), 133.0(C), 131.7(CH), 118.1(CH), 71.5(CH), 71.3(CH),
70.9(CH);67.6(CH₂), 49.0(CH₂), 48.99(CH₂), 32.1(CH₂), 29.8(CH₂), 29.7 (CH₂), 29.5(CH₂), 29.4(CH₂), 25.3(CH₂), 22.9(CH₂), 20.8(CH₂), 19.8(CH₃), 14.4(CH₃).

### Example 2: Preparation of isobutyrate of riboflavin

Riboflavin was suspended in isobutyric anhydride while perchloric acid was added drop wisely under room temperature. Then the mixture was stirred for 7h until the reaction mixture turned to be mauve. Ethyl ether was added to precipitate and crystals were obtained by filtering. The crystals were dissolved in 30ml n-butanol and n-butanol was then washed out by excess water. Next, the crystals were again precipitated by adding ethyl ether and filtrating. The crude product was obtained by vacuum-drying and filtrating. The target compound was obtained by using the separation methods described in Example 1.

The melting point of this compound is 162-166°C. The result of element analysis was N8.41 (calculated values,%) and N8.32 (measured values, %).

### Example 3: Preparation of 2,6-dimethoxybenzoate of riboflavin 5g of riboflavin and 11g of 2,6-dimethoxybenzoylchloride were weighed in an Erlenmeyer flask, then reacted at 80-90°C for 1h with stirring. The mixture was cooled and 10ml methanol was added slowly. The mixture was added into 2L water and yellow crystals were precipitated. The crystals were filtrated, washed by water and dried to obtain crude product of about 11.4g. The crude product was dissolved in 80ml pyridine and filtrated. The filtration was put into 2L water to form crystals. The obtained crystals were dried and weighed 7.8g. The target compound was obtained by using the methods described in Example 1.

The melting point of this compound is 162-166°C. The result of element analysis was C61.60,H5.09,N5.42 (calculated values,%) and C61.41,H5.24,N5.29 (measured values, %).

### Example 4: Preparation of adamantane acid ester of riboflavin.

In a container with adamantane acyl chloride, 1g of riboflavin and 20ml of pyridine were added, and then heated while stirring constantly until reflux slowly. The reflux was kept on continuously for another 2 hours and the reaction mixture was cooled overnight after all the riboflavin was gradually dissolved. The unreacted riboflavin was filtered and pyridine was removed by distilling under reduced pressure. The residue was dissolved in methanol. Then active carbon was added to decolor and filtrated. Crystals were obtained by pouring the filtration into water. After cooling and filtrating, the crude product was obtained. The crude product was then dissolved in methanol, crystallized coldly, filtrated and dried. The target compound was obtained by using the methods described in Example 1.

The melting point of this compound is 118-120°C. The result of element analysis was N5.82 (calculated values,%) and N5.45 (measured values, %).

### Example 5: Preparation of the w/o suspending preparation in which the compound of the invention is the active substrate

Recipe:

| | |
|---|---|
| Compound of Formula II | 50 mg (or 50-150mg) |
| ethyl oleate | 0.5 ml(or 0.1-1 ml) |
| camellia oil | 0.5 ml(or 0-1ml) |

The compound of Formula II was smashed to 2-10µ by gas stream and mixed with ethyl oleate and camellia oil. The preparation was put in 1ml ampoule and sterilized to obtain the w/o suspending preparation.

### Example 6 Long-acting effect of the riboflavin monoester in rats

Male wistar Rats were used in the animal experiments to test the effects of the Long-acting riboflavin in the prevention of ariboflavinosis. The urinary riboflavin excretion and blood glutathione reductase activity coefficient and the content of riboflavin in plasma were measured.

### Materials and methods

### 1. Feeding and grouping of the animals

60 Wistar rats weighing 64.5±4.6g were used in the experiment. The rats were raised individually in metabolic cages. Before the experiment, AIN-76 diet was fed to the animal for one week for adaptation. The rats were randomly assigned into six groups: control group, riboflavin deficiency group, riboflavin group, low dosage Long-acting riboflavin monoester (LRM) group, medium dosage LRM group and high dosage LRM group. In the experiment, AIN-76 diet deficient in riboflavin was given, and tap water was provided the daily food-intake was recorded. The rats were weighed every 6 days. At the beginning of the experiment, orbital plexus blood from the control group was sampled and anticoagulant was added. The blood glutathione reductase activity coefficient (BGRAC) was measured. The plasma was obtained by centrifuging the blood and the riboflavin content was measured. Urine was collected at the 3^{rd} day and the urinary riboflavin excretion was measured. At the 12^{th}, 30^{th}, 60^{th} and 90^{th} days of the experiment, orbital plexus blood and urine from each of the other five groups were sampled, BGRAC was measured, content of riboflavin in plasma and urine were measured respectively.

To the riboflavin-free diet, the riboflavin-free vitamin mixture was used, and casein and starch were treated by immersing in 1% sodium bisulphate solution for 24h, and then washing and drying. The treated casein and starch were analyzed at Beijing Institute of Nutrition and Food Safety to ensure that the riboflavin was destroyed.

### 2. The long-acting riboflavin monoester (LRM) and the dosage for Injection

The LRM (75mg/ml) was intramuscularly injected to the right back leg of the animal after diluted to the appropriate concentration in the mixture oil. The dosage recommended by the NAS-NRC, That is 5.01g LRM for 90 days per rat, was applied to the medium LRM group. For the low or high LRM groups, the dosage was halved or doubled. The riboflavin group animals were injected the w/o suspending preparation of riboflavin, which was made by grinding riboflavin and mixture oil in an agate mortar. The dosage was the same as that used in the medium dosage LRM group. For the riboflavin deficiency group, the same volume of mixture oil without any riboflavin was injected.

### Results

### 1. The general condition, food intake and body weight

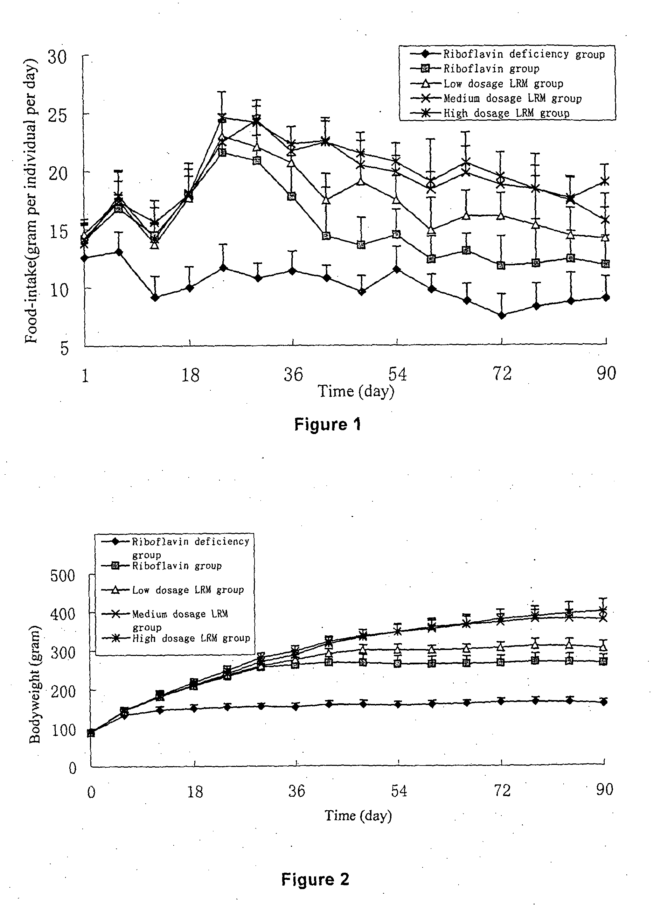

Two rats in the riboflavin deficiency group died of anaesthesia during blood sampling at the 60^{th} day of the experiment. One rat in the medium dosage LRM group died of diarrhea in the 2^{nd} week. The food intake of the riboflavin deficiency group was significantly lower than that of other groups from the very beginning and was maintained at about 10g per rat per day thereafter. Most of the rats in this group showed depilation and some suffered diarrhea .In the riboflavin group, the food intake did not drop until 25 days later and still more than that in the riboflavin deficiency group. The food intake of the three LRM groups was higher than the riboflavin group and the riboflavin deficiency group. However, after 30 days, the food-intake of the low dosage LRM group decreased. The medium and high dosage LRM groups did not show much difference in food intake until the last few days. Also in the last few days, the food intake of the medium dosage LRM group decreased and was lower than that of the high dosage LRM group (Figure 1). The change in body weight was consistent with the food-intake change. At the end of the experiment, the gain of body weight in the medium dosage LRM group was lower than that of the high dosage LRM group (Figure 2).

### 2. Urinary riboflavin excretion

As in Table 1, in the riboflavin deficiency group, the riboflavin excreted in urine significantly decreased at the 15^{th} day and fluctuated at a very low level afterwards. The changes in the riboflavin group, low dosage LRM group and medium dosage LRM group were similar to that in the riboflavin deficiency group, except that the riboflavin discharged in urine in these three groups was higher than that in the riboflavin deficiency group after 15 days. In the high dosage LRM group, the urinary riboflavin excretion markedly increased at the 15^{th} day and then rapidly decreased after the 30 days, while the urinary riboflavin excretion was still higher than that in the other groups.

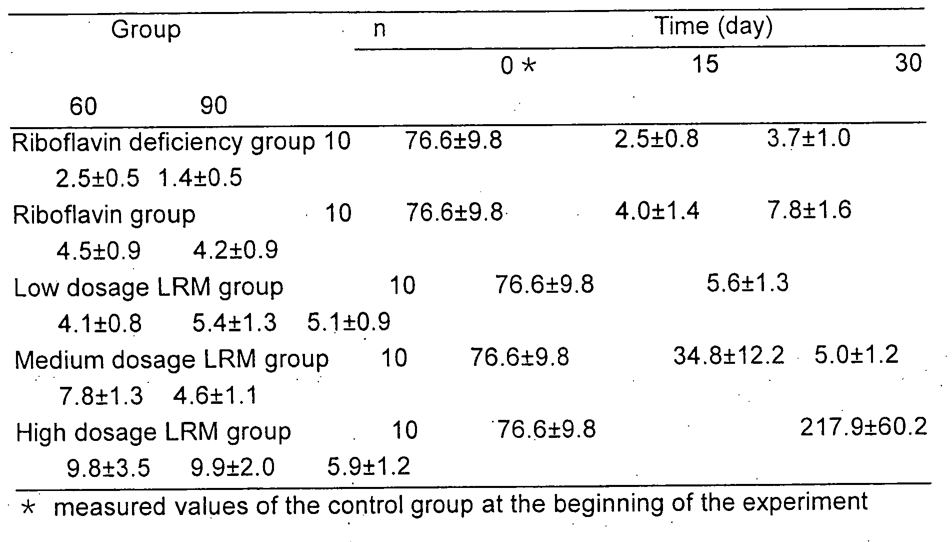

### 3. BGRAC

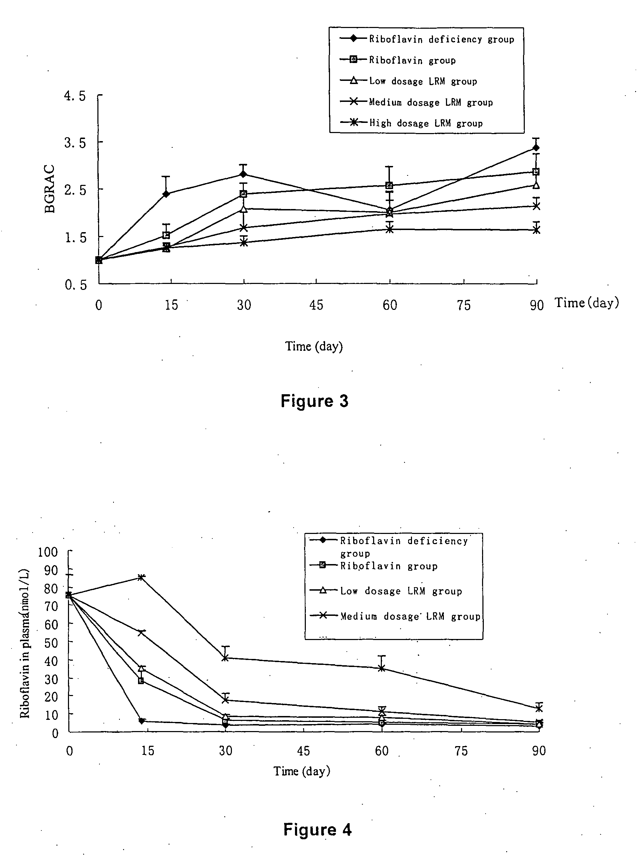

In the riboflavin deficiency group, the BGRAC significantly increased at the fifteenth (15^{th}) day and decreased due to unknown reasons after 60 days; In the riboflavin group, the BGRAC gradually increased and significantly higher than that in the LRM groups. and lower than that in the riboflavin deficiency group during the first 30 days; The BGRAC in the LRM groups was also elevated, in the order as follows the low dosage LRM group > the medium dosage LRM group > the high dosage LRM group (Figure 3).

### 4. The content of riboflavin in plasma

All groups except for the high dosage LRM group showed decline in the content of riboflavin in plasma (Figure 4), in the order as follows: riboflavin deficiency group > riboflavin group > low dosage LRM group > medium dosage LRM group (especially during the first 30 days). In the high dosage LRM group, the riboflavin content in plasma was first elevated and then declined. HPLC was applied to detect the FAD, FMN and riboflavin. However, FAD and FMN were not detected in the plasma of most rats except for a few samples from the normal control group and the high dosage LRM group.

### Conclusions

1. After the rats were fed with riboflavin-free diet, the plasma riboflavin level rapidly declined, while the riboflavin excreted in urine decreased and the BGRAC elevated. At the same time, the food intake was lowered and the body growth stopped.
2. The w/o suspending preparation of riboflavin had some preventive effect on riboflavin deficiency by injection. But the long-acting effect was not as good as the w/o suspending preparation of the LRM at the same dosage.
3. In the medium and high dosage LRM groups, the animals gained weight significantly. However, after a few days, the plasma riboflavin content, the urinary riboflavin excretion declined and the BGRAC required increased. This fact indicated that the riboflavin they took from the normal diet was higher than for normal physiological activities, thus the excess riboflavin was discharged in urine. The medium and high LRM injected could slowly release free riboflavin and meet the demand of the body. At the later stage of the experiment, little riboflavin could be detected in the urines of these two groups.

### Example 7: Effect of the long-acting riboflavin in the prevention of oral ulcer after chemotherapy.48 leukemia cases aged 3-11 years, 32 males and 16 females were recruited. 35 cases of acute lymphocytic leukemia and 13 cases of acute nonlymphocytic leukemia (6 M₂ cases, 3 M₃ cases, 2 M₄ cases and 2 M₅ cases) were also included.

Treatment: Regular chemotherapies, such as CODPL, HDMTX , DA were adopted. Other chemotherapies regimens were not listed. Tetrahydrofolate folic calcium was used to against the toxicity of HDMTX (hige-dose MTX). Hydration and alkalinizing of urine were performed in all patients by Dobll s solution. 5% NaHCO₃ and 3% peroxide hydrogen were used to rinse the mouth alternatively. Part of patients was injected intramuscularly with long-acting riboflavin at dose of 150mg before regular chemotherapy was initiated.

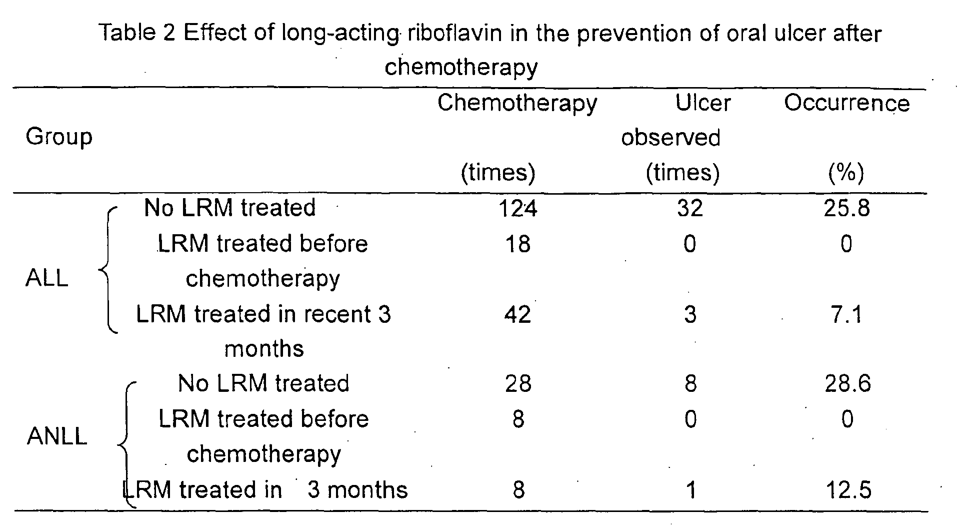

The results indicated that LRM treatment prior to chemotherapy could effectively prevent the occurrence of oral ulcer after chemotherapy. The LRM treatment also could effectively decrease the incidence of oral ulcer within 3 months after chemotherapy.

### Example 8: Effect of long-acting riboflavin monoester in the prevention of catarrh after bone marrow transplantation for leukemia treatment.

28 cases of leukemia with bone marrow transplantation were recruited. 150mg LRM was injected for prevention of catarrh. Only 4 cases (14.3%) suffered oral ulcer. In the control, not treated by LRM, 4 of the 6 cases suffered serious oral ulcer, case developed whole periodontal necrosis.

### Example 10: Effect of long-acting riboflavin in the treatment of refractory oral ulcer.

Mr. Zhang, a 35-year-old man, had suffered several small to large oral ulcers since the year 1984. The local hospital diagnosed it as "aphthae". Though he orally took riboflavin and used local external layers, the situation was not improved. The ulcer occurred at intervals of about 4 months and was exacerbated by fatigue. From the year 1995, he began to take Chinese traditional medicines and the state of ulcer was controlled to some extent. However, the ulcer constantly reoccurred after heavy work. The ulcers generally numbered 3 to 4 and the location was not fixed.

In the year 1999, Mr. Zhang was injected with the w/o suspending preparation of LRM. Though he felt fatigue and slept little sometimes, the oral ulcer had not occurred ever since.

### Medical applicability

The compounds and preparations mentioned in the invention can be widely used in treatment of many diseases, such as catarrhs caused by bone marrow transplantation for leukemia and chemotherapy , oral and digestive tract catarrh, ariboflavinosis, persistent oral ulcer, coronary heart disease and hypertension syndrome, arthritis and burn wound.

## Claims

1. A compound of riboflavin derivatives, comprising all ester derivatives of riboflavin or hydroxyl group of ribitol on the lateral chain of sulforiboflavin except the tetra-stearate ester, tetra-palmitate ester, tri-laurate ester, tetra-decanoate ester, tetra-butyrate ester, tetra-acetate ester, tetra-tryptophan ester, phosphate ester of riboflavin.

2. The compound according to claim 1, wherein the riboflavin derivatives are the mono-, di-, tri- or tetra-esters of the compound of the following Formula (I), 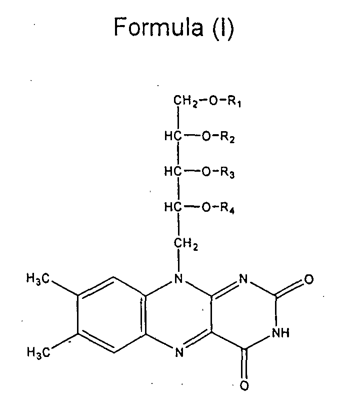 in which the groups of R₁, R₂, R₃, R₄ may be different, identical or independent to each other.

3. The compound according to claim 2, wherein the lauric acid monoester of the following Formula (II) is formed by R1 being a lauroyl group, R2, R3 and R4 all being hydrogens of Formula (II)

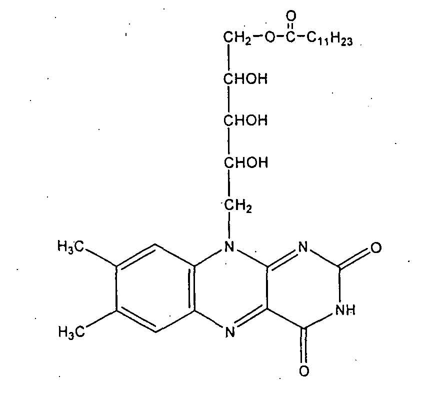

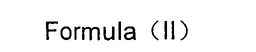

4. A synthesis process of preparation of the compound of claim 3, wherein lauroyl chloride is added drop wisely to riboflavin or sulphoriboflavin suspending in polar organic solvent.

5. The process according to claim 4, wherein the polar organic solvent suspending riboflavin or sulphoriboflavin is pyridine or N, N-dimethylformamide.

6. A w/o suspending preparation using the compound of claim 3 as a main active constituent being mainly composed of ethyl oleate and the compound in Formula II.

7. The w/o suspending preparation according to claim 6, wherein camellia oil is added and the ratio of weight and volume of each ingredient are as follows:
| | |
|---|---|
| Compound II | 50-150 mg |
| Ethyl oleate | 0.1-1 ml |
| Camellia oil | 0-1 ml |

8. The w/o suspending preparation according to claim 7, wherein the preferable ratio of weight and volume of each ingredient are as follows:
| | |
|---|---|
| Compound of Formula II | 150 mg |
| Ethyl oleate | 0.5 ml |
| Camellia oil | 0.5 ml |

9. An application of the compound of any one of claims 1 to 3, wherein the compound can be used in preparing the medicament for ariboflavinosis.

10. An application of the compound of any one of claims 1 to 3, wherein the compound can be used in preparing the medicament for digestive tract catarrh caused by bone marrow transportation, leukemia or chemotherapy.

11. An application of the compound of any one of claims 1 to 3, wherein the compound can be used in preparing the medicament for persistent oral ulcer.

12. An application of the compound of any one of claims 1 to 3, wherein the compound can be used in preparing the medicament for coronary heart disease, hypertension syndrome, arthritis and burn wound.
